# DEMANDE DE BREVET EUROPEEN

(11) **EP 4 285 941 A1**
(43) Date de publication de la demande: **06.12.2023**
(21) Numéro de dépôt: 23175906.9
(22) Date de dépôt: 29.05.2023
(51) Int. Cl.: A61L 9/22, F24F 8/192, F24F 8/30

(54) **DISPOSITIF IONIQUE DE TRAITEMENT DE L'AIR**

(30) Priorité: 30.05.2022 FR 2205174
(71) Demandeur: Teqoya, 33730 Villandraut (FR)
(72) Inventeur: GUITTON, Pierre, 75020 PARIS (FR); BRETON, Jacques, 33600 PESSAC (FR); MARCHAND, Renaud, 33730 VILLANDRAUT (FR)
(74) Mandataire: Touroude & Associates

(57) **Abrégé**

L'invention concerne un dispositif ionique de traitement (500) de particules comprenant :
- une source électrique ;
- une carter (1) formant un conduit (11) s'étendant le long d'un axe longitudinal (550) et le long duquel se propage un flux d'air comportant des particules à filtrer,
- un étage d'ionisation configuré pour générer un champ électrique afin de produire un plasma formé de l'air et des particules se propageant dans le conduit (11), l'étage d'ionisation comportant une pluralité d'ioniseurs (2),
- un étage collecteur configuré pour collecter les particules ionisées par l'étage d'ionisation. Le dispositif de filtration (500) selon l'invention comporte un organe attracteur (4) configuré pour attirer vers lui les particules ionisées par l'étage d'ionisation, l'organe attracteur (4) étant situé dans une position intermédiaire entre les ioniseurs (2) de l'étage d'ionisation.

## Description

Le contexte technique de la présente invention est celui du bâtiment. Plus particulièrement, l'invention se rapporte au dépoussiérage et à la dépollution de l'air entrant et/ou circulant dans un local fermé tel que, par exemple, une pièce d'un logement.

Dans l'état de la technique, on connait des systèmes de dépollution de l'air intérieur d'une pièce d'un logement mettant en oeuvre, par exemple, des ioniseurs d'air, qui réalisent un piégeage des particules et poussières, et/ou ou une désactivation des bio-contaminants, présents dans l'air ambiant de la pièce ou entrant dans celle-ci.

De tels systèmes connus de dépollution de l'air intérieur mettent en oeuvre une ionisation localisée dans le flux d'air circulant au travers desdits systèmes de dépollution, entre une face d'entrée et une face de sortie. Plus particulièrement, la génération d'un champ électrique fort localisé permet d'ioniser localement l'air. Les ions ainsi produits se diffusent alors dans ledit flux d'air et polarisent électriquement les particules présentes dans le flux d'air.

Un inconvénient connu de tels systèmes de dépollution réside dans l'hétérogénéité de la densité des ions dans le flux d'air traversant de tels systèmes de dépollution. En effet, malgré la présence de plusieurs ioniseurs répartis dans de tels systèmes de dépollution, il demeure difficile de produire un champ de densité ionique uniforme au travers du système de dépollution, conduisant à des hétérogénéités de polarisation électrique des particules présentes dans le flux d'air et, in fine, des inhomogénéité de traitement du flux d'air. De tels inhomogénéité conduisent finalement à pénaliser l'efficacité de tels systèmes de dépollution.

La présente invention a pour objet de proposer un nouveau système de dépollution de l'air intérieur d'un local mettant en oeuvre une pluralité d'ioniseurs d'air et permettant d'obtenir une homogénéité maximale de l'ionisation de l'air traité.

Un autre objet de la présente invention est d'améliorer l'efficacité de l'ionisation du flux d'air traversant un tel système de dépollution.

Pour ce faire, l'invention a pour objet un dispositif ionique de traitement d'un air chargé de particules, le dispositif ionique de traitement électronique comprenant :
- une source électrique,
- un carter formant un conduit s'étendant le long d'un axe longitudinal et le long duquel se propage un flux d'air comportant des particules à filtrer,
   - un étage d'ionisation configuré pour charger électriquement des particules se propageant dans le conduit, dites particules ionisées, l'étage d'ionisation comportant une pluralité d'ioniseurs polarisés par la source électrique.

Selon l'invention, le dispositif ionique de traitement comporte un organe attracteur configuré pour attirer vers lui les particules ionisées par l'étage d'ionisation, l'organe attracteur étant situé dans une position intermédiaire entre les ioniseurs de l'étage d'ionisation.

Dans le contexte de la présente invention, la polarisation de l'étage d'ionisation par la source électrique s'entend comme une polarisation électrique, la source électrique étant reliée électriquement à chaque ioniseurs afin de fournir une énergie électrique nécessaire à leur fonctionnement.

Dans le contexte de l'invention, le dispositif ionique de traitement de l'air est soit du type d'un dispositif ionique d'ionisation, soit du type d'un dispositif ionique de filtration. Dans ce cas, le dispositif ionique de traitement comporte alors un étage collecteur configuré pour collecter les particules ionisées par l'étage d'ionisation, l'étage collecteur étant polarisé par la source électrique. Dans le contexte de la présente invention, la polarisation de l'étage collecteur par la source électrique s'entend comme une polarisation électrique, la source électrique étant reliée électriquement à l'étage collecteur afin de fournir une énergie électrique nécessaire à son fonctionnement.

Ainsi, dans le contexte de l'invention, lorsque le dispositif ionique de traitement est du type d'un dispositif ionique d'ionisation, le dispositif ionique d'ionisation est configuré pour ioniser les particules traversant le conduit, de sorte que, en sortie du dispositif ionique de traitement, l'air transporte une grande quantité d'ions.

A contrario, dans le contexte de l'invention, lorsque le dispositif ionique de traitement est du type d'un dispositif ionique de filtration, l'étage collecteur situé en aval de l'étage d'ionisation permet de capter les particules ainsi chargées électriquement de sorte que, en sortie du dispositif ionique de traitement, le flux d'air sortant dudit dispositif ionique de traitement et formant l'air ambiant est exempt de telles particules.

Il faut tout d'abord comprendre ici que le conduit formé par le carter du dispositif ionique de traitement selon l'invention est délimité par les parois du carter : en d'autres termes, le flux d'air contenant les particules à filtrer se propage à l'intérieur du volume dudit conduit délimité par des parois du carter du dispositif ionique de traitement selon l'invention, et cette propagation s'effectue selon une direction principale sensiblement parallèle, à d'éventuelles turbulences près, à l'axe longitudinal du carter et du conduit précités.

Dans le contexte de l'invention, les ioniseurs sont par exemple orientés perpendiculairement ou sensiblement perpendiculairement par rapport à l'axe longitudinal. Bien entendu, la présente invention couvre toute les orientations possibles des ioniseurs, relativement à l'axe longitudinal.

Par ailleurs, il faut également comprendre ici que les ioniseurs sont répartis sur une périphérie des parois précitées du carter du dispositif ionique de traitement. En d'autres termes, les ioniseurs sont disposés, au sein du conduit délimité par les parois extérieures du carter, le long d'un pourtour d'une section de ce conduit selon un plan sensiblement perpendiculaire à l'axe longitudinal précédemment défini. Cette configuration avantageuse permet ainsi de libérer un espace central du conduit, facilitant ainsi l'écoulement dudit flux d'air au travers du dispositif ionique de traitement. En outre, cette configuration permet avantageusement de faciliter l'accès aux ioniseurs et, le cas échéant, à l'étage collecteur lorsque le dispositif ionique de traitement est du type d'un dispositif ionique de filtration. Enfin, cette configuration permet de faciliter la fixation mécanique des différents composants du dispositif ionique de traitement selon l'invention, et notamment de la source électrique.

Selon un mode de réalisation, selon une direction parallèle à l'axe longitudinal précité, les ioniseurs sont avantageusement tous situés sensiblement, aux tolérances de fabrication et de montage près, à une même distance d'une extrémité longitudinale du carter du dispositif ionique de traitement selon l'invention. Selon un autre mode de réalisation, le dispositif ionique de traitement selon l'invention comporte plusieurs étages d'ionisation, chaque étage d'ionisation comportant plusieurs ioniseurs répartis en périphérie des parois du carter, de sorte que lesdits ioniseurs sont disposés, au sein du conduit, le long d'un pourtour d'une section de ce conduit et définie selon un plan sensiblement perpendiculaire à l'axe longitudinal. En outre, les différents étages d'ionisation sont répartis au sein du dispositif ionique de traitement le long de l'axe longitudinal.

Selon un exemple de mise en oeuvre de l'invention, les ioniseurs sont dirigés strictement perpendiculairement par rapport à l'axe longitudinal du carter du dispositif ionique de traitement.

Selon un autre exemple de mise en oeuvre de l'invention, les ioniseurs sont dirigés selon une direction formant un angle non nul avec un plan perpendiculaire à l'axe longitudinal du carter et du conduit délimité par les parois de celui-ci, préférentiellement inférieur à 45 degrés environ.

Avantageusement, les ioniseurs de l'étage d'ionisation sont répartis et s'étendent selon au moins deux plans dont un axe directeur est parallèle à l'axe longitudinal. En d'autres termes, l'invention prévoit que les ioniseurs sont avantageusement répartis en au moins deux groupes d'ioniseurs dont chacun s'étend dans un plan contenant une direction parallèle à celle de l'axe longitudinal du carter du dispositif ionique de traitement.

Selon un exemple de mise en oeuvre de l'invention, les au moins deux groupes d'ioniseurs sont sensiblement symétriquement disposés par rapport à l'axe longitudinal du carter, c'est-à-dire, en d'autres termes, qu'ils sont sensiblement placés face à face au sein du conduit délimité par les parois du carter du dispositif ionique de traitement selon l'invention.

Selon l'invention, l'organe attracteur est placé entre les au moins deux groupes d'ioniseurs précités, c'est-à-dire qu'il est placé au sein du conduit délimité par les parois du carter, conduit à l'intérieur duquel se propage le flux d'air contenant les particules à filtrer. En d'autres termes, l'organe attracteur est immergé dans le flux d'air contenant les particules à filtrer.

En attirant les particules ionisées, l'organe attracteur permet d'homogénéiser le flux de ces dernières dans la région du conduit du carter dans laquelle il est placé. En d'autres termes et par analogie mécanique, l'organe attracteur fonctionne comme un ressort étirant, dans sa direction, le plasma formé par les ioniseurs.

Selon différentes caractéristiques du dispositif ionique de traitement selon l'invention, prises séparément ou en combinaison :
- l'organe attracteur s'étend le long de l'axe longitudinal du carter du dispositif ionique de traitement. Plus précisément, l'invention prévoit que l'organe attracteur s'étend selon une direction sensiblement parallèle, aux tolérances de fabrication et de montage près, à la direction de l'axe longitudinal du carter.
- l'organe attracteur est situé au centre du carter. Il faut comprendre ici que l'organe attracteur s'étend sensiblement symétriquement autour de l'axe longitudinal du carter. L'attraction des particules ionisées est ainsi réalisée de manière homogène radialement par rapport à l'axe longitudinal.
- l'organe attracteur est situé en regard des ioniseurs de l'étage d'ionisation. Il faut comprendre ici par "en regard" qu'une particule ionisée par l'un des ioniseurs du dispositif ionique de traitement et se propageant, à partir de l'ioniseur considéré, en direction de l'axe longitudinal selon une direction sensiblement radiale, heurte nécessairement, dans son parcours, l'organe attracteur. En d'autres termes, un plan sensiblement perpendiculaire à l'axe longitudinal et contenant les ioniseurs de l'étage d'ionisation présente une intersection non vide avec l'organe attracteur.
- l'organe attracteur est, selon la direction de l'axe longitudinal, partiellement ou totalement décalé par rapport aux ioniseurs de l'étage d'ionisation. Dans ce cas, et de manière particulièrement avantageuse, l'invention prévoit que l'organe attracteur est décalé par rapport aux ioniseurs de sorte à être placé en amont des ioniseurs relativement au flux d'air, afin de pouvoir étirer et homogénéiser un nuage de particules ionisées au sein du conduit.
- l'organe attracteur prend la forme d'une plaque qui s'étend, dans le conduit précédemment défini, en regard des ioniseurs de l'étage d'ionisation. Selon différents exemples de réalisation de cette forme particulière, une telle plaque peut être sensiblement plane ou présenter une forme courbe.
- l'organe attracteur prend la forme d'un cylindre qui s'étend, dans le conduit, le long de l'axe longitudinal. Selon un exemple particulièrement avantageux d'une telle configuration, l'organe attracteur prend la forme d'un fil matérialisant, aux tolérances de fabrication et de montage, l'axe longitudinal du carter du dispositif ionique de traitement selon l'invention.
- selon la direction de l'axe longitudinal, l'organe attracteur s'étend entre un bord inférieur et un bord supérieur des ioniseurs de l'étage d'ionisation. Les dénominations "inférieur" et "supérieur" sont ici définies en relation avec le sens de circulation du flux d'air contenant les particules à filtrer dans le conduit du dispositif ionique de traitement selon l'invention. Plus précisément, le terme "inférieur" est à comprendre comme l'amont relativement à ce sens de circulation, et le terme "supérieur" est à comprendre comme l'aval relativement à ce sens de circulation. En d'autres termes, le flux d'air contenant les particules à filtrer circule, au sein du conduit délimité par les parois du carter du dispositif ionique de traitement, selon la direction de l'axe longitudinal, entre une extrémité longitudinale inférieure et une extrémité longitudinale supérieure dudit conduit.
- selon la direction de l'axe longitudinal, un bord inférieur de l'organe attracteur est situé en amont d'un bord inférieur des ioniseurs de l'étage d'ionisation. Avantageusement, une distance, mesurée selon la direction de l'axe longitudinal entre le bord inférieur des ioniseurs et le bord inférieur de l'organe attracteur est inférieure à une dimension mesurée selon la direction de l'axe longitudinal, de l'étage d'ionisation.
- selon la direction de l'axe longitudinal, un bord supérieur de l'organe attracteur est situé en aval d'un bord supérieur des ioniseurs de l'étage d'ionisation. Avantageusement, une distance, mesurée selon la direction de l'axe longitudinal, entre le bord supérieur des ioniseurs et le bord supérieur de l'organe attracteur est inférieure à une dimension, mesurée selon la direction de l'axe longitudinal, de l'étage d'ionisation.
- l'organe attracteur est formé dans un matériau antistatique. D'une manière générale, l'organe attracteur est formé dans un matériau faiblement conducteur électrique.
- le matériau constituant l'organe attracteur comporte un plastique, par exemple un polypropylène, et une charge de carbone. Avantageusement, la quantité de carbone que comprend le matériau formant l'organe attracteur est comprise entre 5% et 20% d'une masse de plastique composant le matériau de l'organe attracteur.
- l'organe attracteur est configuré pour qu'un courant de décharge s'écoulant dans ledit organe attracteur soit inférieur ou égal à 1µA, en particulier pour un système traitant un débit d'air d'environ 100 m³/h. Complémentairement, dans ce cas, une différence de potentiel électrique entre une masse électrique et l'organe attracteur est inférieure ou égale à quelques dizaines de Volts, par exemple 50 V.
- une distance entre les ioniseurs et l'organe attracteur est comprise entre 50 mm et 100 mm. D'une manière générale, la distance entre les ioniseurs et l'organe attracteur est inférieure à 100 mm. Dans le contexte de l'invention, la distance entre les ioniseurs et l'organe attracteur est ajustée en fonction du matériau de l'organe attracteur et de la puissance des ioniseurs ;
- l'organe attracteur est relié à une masse électrique du dispositif ionique de traitement selon l'invention. Avantageusement, le carter du dispositif de filtration est également relié à une masse électrique du dispositif ionique de traitement.
- le carter du dispositif ionique de traitement est réalisé dans un matériau métallique.

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :
[Fig.1] illustre schématiquement en perspective un exemple de réalisation d'un dispositif ionique de traitement selon l'invention,
[Fig.2] est une vue schématique d'un exemple de réalisation d'un dispositif ionique de traitement tel que celui illustré par la figure 1, en perspective coupée selon un plan contenant l'axe longitudinal précédemment défini,
[Fig.3] est une vue schématique en perspective rapprochée d'une extrémité, selon la direction longitudinale précédemment définie, d'un dispositif ionique de traitement tel que celui illustré par les figures 1 et 2,
[Fig.4] est une vue schématique de dessus d'un dispositif ionique de traitement tel que celui illustré par les figures 1 à 3, en coupe selon un plan perpendiculaire à l'axe longitudinal précédemment évoqué,
[Fig.5] est une vue schématique de côté d'un dispositif ionique de traitement tel que celui illustré par les figures 1 à 4, en coupe selon un plan identique au plan de coupe de la figure 2.

Bien entendu, les caractéristiques, les variantes et les différentes formes de réalisation de l'invention peuvent être associées les unes avec les autres, selon diverses combinaisons, dans la mesure où elles ne sont pas incompatibles ou exclusives les unes des autres. On pourra notamment imaginer des variantes de l'invention ne comprenant qu'une sélection de caractéristiques décrites par la suite de manière isolées des autres caractéristiques décrites, si cette sélection de caractéristiques est suffisante pour conférer un avantage technique ou pour différencier l'invention par rapport à l'état de la technique antérieur.

En particulier toutes les variantes et tous les modes de réalisation décrits sont combinables entre eux si rien ne s'oppose à cette combinaison sur le plan technique.

Sur les FIGURES, les éléments communs à plusieurs FIGURES conservent la même référence.

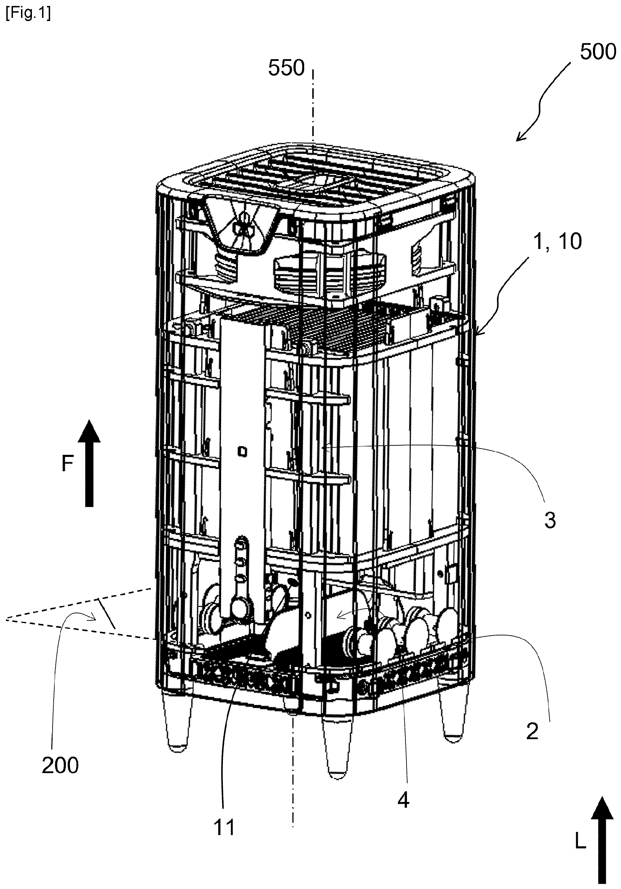

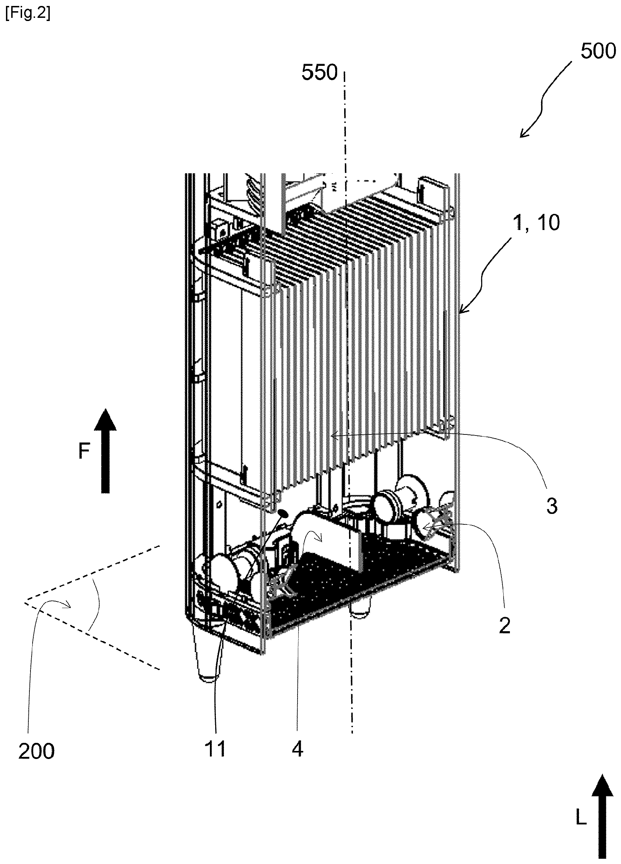

Les FIGURES 1 et 2 montrent schématiquement en perspective un exemple de réalisation d'un dispositif ionique de traitement 500 selon l'invention, la FIGURE 2 montrant plus particulièrement une perspective coupée du dispositif illustré par la FIGURE 1.

En référence à ces FIGURES, un dispositif ionique de traitement 500 selon l'invention comprend, notamment, un carter 1 dont les parois 10 délimitent un conduit 11 dans lequel circule un flux d'air contenant des particules à filtrer. La circulation du flux d'air au sein du conduit 11 est illustrée par la flèche F sur la FIGURE 1. Sur les FIGURES 1 et 2, les parois 10 du carter 1 sont représentées transparentes pour plus de visibilité des éléments reçus dans le conduit 11 précité. Avantageusement, le carter 1 est réalisé dans un matériau métallique, par exemple par pliage d'une tôle mince, et il est électriquement relié à une masse électrique du dispositif ionique de traitement 500.

Selon l'exemple plus particulièrement illustré ici, le carter 1 présente sensiblement la forme d'un parallélépipède rectangle dont les arêtes sont arrondies et dont une section perpendiculaire à sa plus grande dimension d'extension est sensiblement carrée.

En référence à ce qui précède, la direction de la plus grande dimension d'extension du carter 1 sera désignée dans ce qui suit comme direction longitudinale L du carter 1, du conduit 11 et du dispositif ionique de traitement 500. En référence à la direction longitudinale L, on définit comme axe longitudinal 550 du carter 1, du conduit 11 et du dispositif ionique de traitement 500 l'axe reliant entre eux les points d'intersection des diagonales des sections du carter 1 prises perpendiculairement à la direction longitudinale L précitée. En référence à ces directions et orientations, la vue représentée en figure 2 est une vue en perspective coupée selon un plan contenant l'axe longitudinal 550 précité.

Dans le cas, non illustré par les FIGURES, où le carter 1 présente une forme sensiblement cylindrique, l'axe longitudinal 550 est confondu avec l'axe de révolution de la forme cylindrique du carter 1.

En référence à la direction longitudinale L précitée et au sens de circulation du flux d'air contenant les particules à filtrer, illustré par la flèche F sur les FIGURES 1 et 2, on désignera dans ce qui suit comme "inférieure" l'extrémité longitudinale du carter 1 et du dispositif ionique de traitement 500 située par laquelle le flux d'air précité entre dans le conduit 11, et on désignera dans ce qui suit comme "supérieure" l'extrémité longitudinale du carter 1 et du dispositif ionique de traitement 500 par laquelle le flux d'air précité sort du conduit 11 et du dispositif ionique de traitement 500. En d'autres termes, l'extrémité longitudinale inférieure et l'extrémité longitudinale supérieure du dispositif ionique de traitement 500 représentent respectivement l'amont et l'aval de la circulation du flux d'air contenant les particules à filtrer dans le conduit 11.

Comme le montrent les FIGURES 1 et 2, le dispositif ionique de traitement 500 comprend également, logés au sein du conduit 11 précédemment défini, une pluralité d'ioniseurs d'air 2, ainsi qu'une pluralité de plaques de collection 3.

Chaque ioniseur 2 est polarisé par une source électrique non visible sur la FIGURE 1, de manière à créer un plasma dans le flux d'air qui circule au sein du conduit 11 afin de charger négativement les particules contenues dans ce flux d'air.

Chaque plaque de collection 3 est également polarisée par la source électrique précédemment évoquée, de telle manière que les particules chargées négativement dans le plasma créé par les ioniseurs 2 sont attirées vers lesdites plaques de collection 3, réalisant ainsi leur piégeage et, donc la dépollution recherchée du flux d'air qui circule au sein du conduit 11.

Selon l'exemple plus particulièrement illustré par les FIGURES, les ioniseurs 2 sont situés au voisinage de l'extrémité longitudinale inférieure du carter 1, et les plaques de collection 3 sont situées, selon la direction longitudinale L, en aval des ioniseurs 2. Ainsi, lorsque le flux d'air contenant les particules à filtrer entre dans le conduit 11 par l'extrémité inférieure du carter 1, il traverse tout d'abord la partie dudit conduit 11 située entre les ioniseurs 2 : les particules qu'il contient y sont alors ionisées, puis elles sont collectées sur les plaques de collection 3, réalisant ainsi la dépollution recherchée.

Selon l'exemple de réalisation plus particulièrement illustré ici, les ioniseurs 2 sont au nombre de 6 et ils sont répartis en deux groupes comprenant chacun 3 ioniseurs 2, les ioniseurs 2 de chaque groupe étant disposés face à face deux à deux selon une direction perpendiculaire à l'axe longitudinal 550. Plus précisément, les deux groupes d'ioniseurs 2 définissent ensemble un plan 200 sensiblement perpendiculaire, aux tolérances de fabrication et de montage près, à l'axe longitudinal 550.

Selon l'exemple plus particulièrement illustré par les FIGURES, les plaques de collection 3 présentent chacune une forme sensiblement plane et rectangulaire, et elles s'étendent chacune selon un plan contenant l'axe longitudinal 550 du dispositif ionique de traitement 500. Selon cet exemple, les plaques de collection 3 sont disposées sensiblement parallèlement les unes aux autres deux à deux selon une direction d'empilement perpendiculaire à l'axe longitudinal 550, et leur nombre, ainsi que leur épaisseur et leur écartement deux à deux sont définis de telle manière qu'elles occupent, d'une part, une partie du conduit 11 selon la direction longitudinale L et, d'autre part, la totalité de ce conduit 11 dans un plan perpendiculaire à l'axe longitudinal 550. En d'autres termes, les plaques de collection 3 sont disposées de telle manière que la totalité du flux d'air entrant dans le conduit 11 et circulant entre les ioniseurs 2 passe nécessairement dans la partie du conduit 11 qu'elles occupent.

Selon l'invention, le dispositif ionique de traitement 500 comprend également un organe attracteur 4, polarisé par la source électrique précédemment évoquée, et configuré pour attirer les ions présents dans le plasma créé par les ioniseurs 2. Plus précisément, l'invention prévoit que l'organe attracteur 4 est placé, au sein du conduit 11, dans l'espace compris entre les ioniseurs 2. Avantageusement, l'organe attracteur 4 est réalisé dans un matériau antistatique afin de réduire autant que possible la survenue de phénomènes d'arc électrique dans la région du conduit 11 occupée par l'organe attracteur 4. Par sa présence et sa polarisation, l'organe attracteur 4 attire à lui un flux d'ions, agissant, comme précédemment indiqué, comme une forme de ressort décalant vers lui les ions dérivant hors du plasma créé par les ioniseurs 2. Ainsi l'organe attracteur4 décale vers lui le champ de concentration en ions présent dans le conduit 11. Il s'ensuit une homogénéisation du flux d'air ionisé dans la région de l'organe attracteur 4, c'est-à-dire dans une région éloignée des ioniseurs 2 : ceci permet une meilleur homogénéisation du flux d'air ionisé au sein du conduit 11 et, donc, une efficacité accrue du dispositif ionique de traitement 500.

Selon un exemple non exclusif, l'organe attracteur 4 est réalisé dans un matériau plastique contenant une charge de carbone : avantageusement, le matériau plastique constituant l'organe attracteur 4 est un polypropylène dans lequel la charge de carbone représente préférentiellement entre environ 5 et 20% de la masse de plastique, de telle manière qu'un courant de décharge susceptible de s'écouler dans l'organe attracteur 4 est toujours inférieur à quelques fractions d'Ampère, préférentiellement inférieur à 1 micro-Ampère environ.

D'une manière plus générale, et selon un mode de réalisation particulièrement avantageux du dispositif ionique de traitement 500 selon l'invention, une grande majorité des composants du dispositif ionique de traitement 500, et comportant notamment l'organe attracteur 4 et/ou le carter 1, et/ou tout ou partie des parois 10 et/ou une grille d'entrée du dispositif ionique de traitement 500 sont formés d'un tel matériau plastique antistatique. Cette configuration avantageuse permet de faciliter la mise en oeuvre d'une décharge électrostatique desdits composants précités.

Selon l'exemple plus particulièrement illustré par les FIGURES, l'organe attracteur 4 se présente sous la forme d'une plaque sensiblement plane de forme sensiblement rectangulaire, et il s'étend, entre les ioniseurs 2, selon un plan contenant l'axe longitudinal 550.

Plus précisément, et comme le montrent les différentes FIGURES, l'organe attracteur 4 s'étend selon un plan sensiblement parallèle, aux tolérances de fabrication et de montage près, aux parois 10 du carter 1 sur lesquelles sont placées les ioniseurs 2. Autrement dit, l'organe attracteur 4 est placé face aux ioniseurs 2 selon une direction perpendiculaire à celle de l'axe longitudinal 550, chaque groupe d'ioniseurs 2 étant situé en regard d'une face de la plaque constituant l'organe attracteur 4.

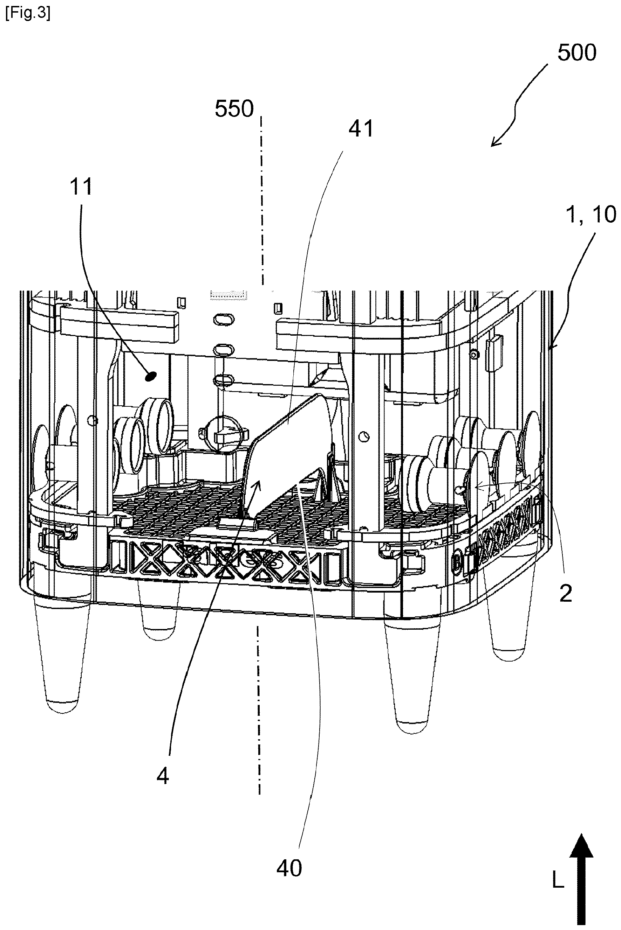

La FIGURE 3 est une vue en perspective rapprochée de la partie du dispositif ionique de traitement 500 dans laquelle sont placés les ioniseurs 2 et l'organe attracteur 4, c'est-à-dire, en référence aux directions et orientations précédemment définies, de l'extrémité inférieure du carter 1 et du conduit 11. On retrouve sur cette figure le carter 1, ses parois 10, le conduit 11, les ioniseurs 2, l'organe attracteur 4, et la flèche F illustrant le sens de circulation d'un flux d'air contenant des particules à filtrer au sein du conduit 11.

Comme le montre plus précisément la FIGURE 3, les bords de l'organe attracteur 4 sont arrondis : ceci permet notamment d'éviter la création d'arcs électriques sur ces bords.

Également, la FIGURE 3 montre de manière plus précise la forme de l'organe attracteur 4 selon l'exemple illustré ici : l'organe attracteur 4 présente la forme générale d'une plaque trapézoïdale dont la plus grande base forme bord inférieur 40 situé du côté de la partie inférieure, précédemment définie, du dispositif ionique de traitement 500, et dont la plus petite base forme bord supérieur 41 situé du côté de la partie supérieure, précédemment définie, du dispositif ionique de traitement 500. En d'autres termes, une dimension, mesurée selon la direction longitudinale L, de l'organe attracteur 4, diminue vers les bords de celui-ci situés les plus proches de parois 10 du carter 1. Ceci permet également de réduire les risques de formation d'arcs électriques entre l'organe attracteur 4 et le carter 1.

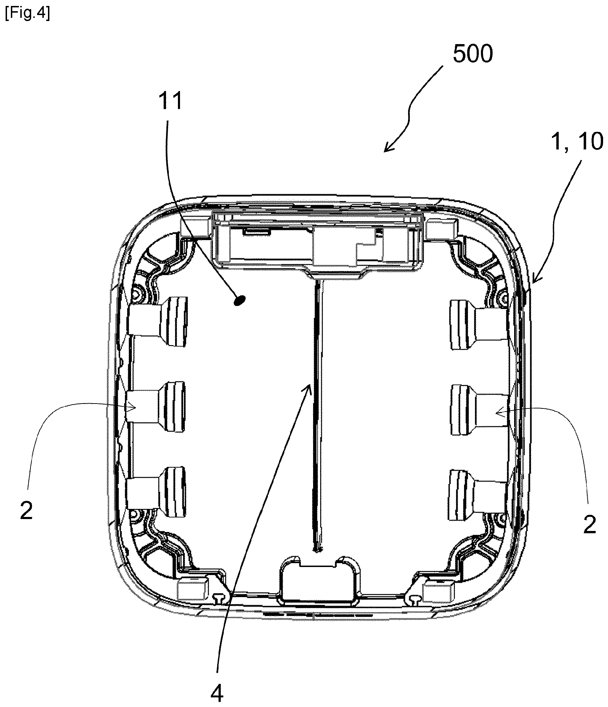

La FIGURE 4 est une vue schématique d'un dispositif ionique de traitement 500 tel que celui illustré par les FIGURES 1 à 3, en coupe selon un plan perpendiculaire à l'axe longitudinal 550 et en vue de dessus, c'est-à-dire en vue depuis l'extrémité supérieure du carter 1 et du conduit 11. On retrouve sur cette figure le carter 1, ses parois 10, les ioniseurs 2 et l'organe attracteur 4.

Comme le montre la FIGURE 4, l'organe attracteur 4 s'étend sensiblement parallèlement aux parois 10 du carter 1 sur lesquelles sont placés les ioniseurs 2, et il est placé en position sensiblement médiane entre ces parois et entre les deux groupes d'ioniseurs 2. En d'autres termes, le plan d'extension de l'organe attracteur 4 selon cet exemple contient l'axe longitudinal 550. Ceci permet une homogénéisation optimale du flux d'air ionisé entre les deux groupes d'ioniseurs 2.

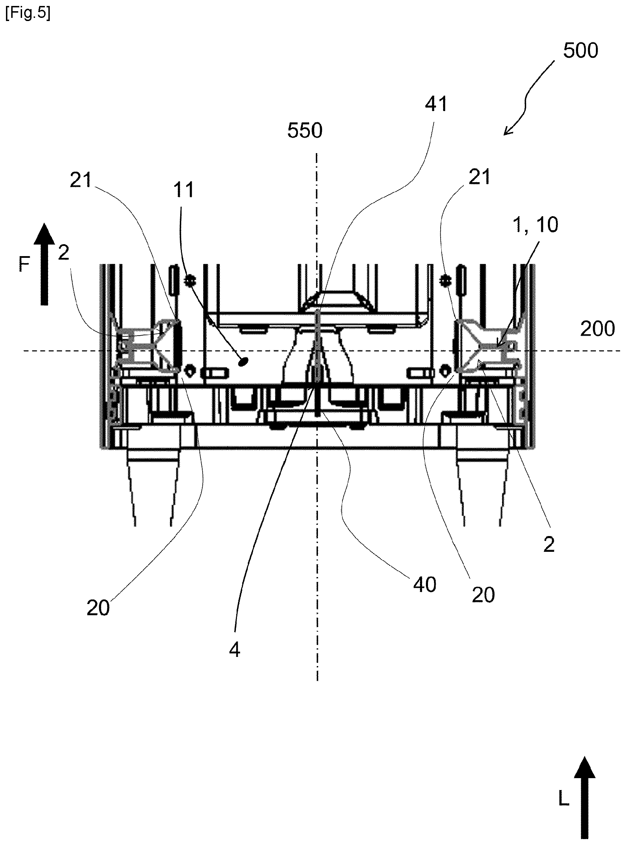

La FIGURE 5 est une vue schématique d'un dispositif ionique de traitement 500 tel que celui illustré par les FIGURES 1 à 4, en coupe selon un plan contenant l'axe longitudinal 550 et en vue de côté. Plus précisément, la FIGURE 5 est une vue depuis une paroi 10 du carter 1 perpendiculaire aux parois 10 du carter 1 sur lesquelles sont placées les ioniseurs 2. On retrouve sur la FIGURE 5 le carter 1, ses parois 10, le conduit 11, les ioniseurs 2 et l'organe attracteur 4.

Comme le montre la FIGURE 5, l'organe attracteur 4 s'étend, selon la direction longitudinale L, de part et d'autre du plan 200, précédemment décrit, défini par les deux groupes d'ioniseurs 2 ensemble. Plus précisément, un bord inférieur 40 de l'organe attracteur 4 est situé au-dessous d'un bord inférieur 20 des ioniseurs 2, c'est-à-dire en amont, relativement au sens F de circulation du flux d'air dans le conduit 11, des ioniseurs 2, et un bord supérieur 41 de l'organe attracteur 4 est situé au-dessus d'un bord supérieur 21 des ioniseurs 2, c'est-à-dire en aval, relativement au sens F de circulation du flux d'air dans le conduit 11, des ioniseurs 2.

Avantageusement, une distance, mesurée selon la direction longitudinale L, entre un bord inférieur 40 de l'organe attracteur 4 et un bord inférieur 20 des ioniseurs 2 est inférieure à une dimension, mesurée selon la direction longitudinale L, des ioniseurs 2. De manière complémentaire, une distance, mesurée selon la direction longitudinale L, entre un bord supérieur 41 de l'organe attracteur 4 et un bord supérieur 21 des ioniseurs 2 est avantageusement inférieure à une dimension, mesurée selon la direction longitudinale L, des ioniseurs 2.

L'invention, telle qu'elle vient d'être décrite et illustrée, permet de réaliser, par des moyens simples, un dispositif ionique de traitement dans lequel l'ionisation d'un flux d'air contenant des particules à filtrer est réalisée de manière homogène dans l'ensemble du volume occupé par ce flux d'air, conduisant à une dépollution efficace dudit flux d'air.

Il est, de plus, à noter que la forme et l'agencement de l'organe attracteur 4 permettent ici un nettoyage facilité du conduit 11 et des plaques de collection 3.

Bien sûr, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

De plus, les formes du carter 1 et de l'organe attracteur 4, ainsi que le nombre et la disposition des ioniseurs 2 peuvent varier sans que cela nuise à l'invention, dès lors que ces différents éléments présentent les caractéristiques décrites dans le présent document.

Ainsi, l'invention s'étend notamment à toute forme de carter 1 dans laquelle un axe longitudinal 550 tel que celui décrit dans le présent document peut être défini. De même l'invention s'étend à tout nombre et à toute répartition des ioniseurs 2 dans la mesure où ceux-ci sont agencés dans le conduit 11 de manière à dégager, entre eux, un espace d'accueil de l'organe attracteur 4.

Enfin, l'invention s'étend à des formes et dimensions d'un organe attracteur 4 différentes de celles décrites et illustrées dans le présent document. En particulier, l'invention s'étend à un organe attracteur 4 se présentant sous la forme d'un fil disposé axialement dans le carter 1 et matérialisant l'axe longitudinal 550 de ce dernier. D'une manière plus générale, l'organe attracteur 4 peut également prendre la forme d'un cylindre dont l'axe est sensiblement, aux tolérances de fabrication et de montage près, confondu avec l'axe longitudinal 550, sans que cela nuise à l'invention. Dans ce mode de réalisation, une section transverse du cylindre formant l'organe attracteur 4, prise dans un plan perpendiculaire à l'axe longitudinal 550, et/ou une élongation dudit cylindre relativement à l'axe longitudinal 550 est telle que tous les ioniseurs 2 sont situés même distance dudit cylindre formant ledit organe attracteur 4, ladite distance étant alors considérée selon une direction radiale et perpendiculaire à l'axe longitudinal 550 et/ou audit cylindre, formant ainsi le plus court chemin entre l'organe attracteur 4 et lesdits ioniseurs 2.

## Revendications

1. Dispositif ionique de traitement (500) de particules, le dispositif ionique de traitement (500) comprenant :
- une source électrique ;
- une carter (1) formant un conduit (11) s'étendant le long d'un axe longitudinal (550) et le long duquel se propage un flux d'air comportant des particules à filtrer,
- un étage d'ionisation configuré pour charger électriquement des particules se propageant dans le conduit (11), dites particules ionisées, l'étage d'ionisation comportant une pluralité d'ioniseurs (2) polarisés par la source électrique,
- des plaques de collection permettant d'attirer et de piéger les particules chargées négativement dans le flux d'air,
**caractérisé en ce que** le dispositif ionique de traitement (500) comporte un organe attracteur (4) configuré pour attirer vers lui les particules ionisées par l'étage d'ionisation, l'organe attracteur (4) étant situé dans une position intermédiaire entre les ioniseurs (2) de l'étage d'ionisation, l'organe attracteur (4) étant situé en regard des ioniseurs (2) de l'étage d'ionisation.

2. Dispositif ionique de traitement (500) selon la revendication précédente, dans lequel l'organe attracteur (4) s'étend le long de l'axe longitudinal (550).

3. Dispositif ionique de traitement (500) selon l'une quelconque des revendications 1 à 2, dans lequel l'organe attracteur (4) prend la forme d'une plaque qui s'étend dans le conduit (11) en regard des ioniseurs (2) de l'étage d'ionisation.

4. Dispositif ionique de traitement (500) selon l'une quelconque des revendications 1 à 2, dans lequel l'organe attracteur (4) prend la forme d'un cylindre qui s'étend dans le conduit (11) le long de l'axe longitudinal (550).

5. Dispositif ionique de traitement (550) selon l'une quelconque des revendications 1 à 4, dans lequel, selon la direction de l'axe longitudinal (550), l'organe attracteur (4) s'étend entre un bord inférieur (20) et un bord supérieur (21) des ioniseurs (2) de l'étage d'ionisation.

6. Dispositif ionique de traitement (500) selon l'une quelconque des revendications 1 à 4, dans lequel, selon la direction de l'axe longitudinal (550), un bord inférieur (40) de l'organe attracteur (4) est situé en amont d'un bord inférieur (20) des ioniseurs (2) de l'étage d'ionisation, relativement à une direction (F) du flux d'air.

7. Dispositif ionique de traitement (500) selon la revendication précédente, dans lequel, relativement à la direction de l'axe longitudinal (550), un bord supérieur (41) de l'organe attracteur (4) est situé en aval d'un bord supérieur (21) des ioniseurs (2) de l'étage d'ionisation, relativement à une direction (F) du flux d'air.

8. Dispositif ionique de traitement (500) selon l'une quelconque des revendications précédentes, dans lequel l'organe attracteur (4) est formé d'un matériau antistatique.

9. Dispositif ionique de traitement (500) selon l'une quelconque des revendications précédentes, dans lequel l'organe attracteur (4) est relié à une masse électrique du dispositif ionique de traitement (500).
